(19) Europäisches Patentamt
European Patent Office
Office européen des brevets

(11) **EP 0 745 579 B1**

(12) **FASCICULE DE BREVET EUROPEEN**

(45) Date de publication et mention
de la délivrance du brevet:
**04.08.1999 Bulletin 1999/31**

(51) Int. Cl.$^6$: **C07C 67/04**, C07C 69/54

(21) Numéro de dépôt: 96400977.3

(22) Date de dépôt: 07.05.1996

(54) **Procédé de fabrication d'acrylate de butyle secondaire par réaction de l'acide acrylique et des isomères de butène**

Verfahren zum Herstellen von sekundairem Butylacrylat durch Reaktion von Acrylsäure mit Butenisomeren

Process for producing secondary butyl acrylate by reaction of acrylic acid and butene isomers

(84) Etats contractants désignés:
**AT BE CH DE DK ES FI FR GB GR IE IT LI LU NL PT SE**

(30) Priorité: **02.06.1995 FR 9506594**

(43) Date de publication de la demande:
**04.12.1996 Bulletin 1996/49**

(73) Titulaire: **ELF ATOCHEM S.A.**
**92800 Puteaux, Hauts-de-Seine (FR)**

(72) Inventeurs:
• **Paul, Jean-Michel**
**57070 Metz (FR)**
• **Samuel, Yves**
**57500 Saint-Avold (FR)**
• **Esch, Marc**
**57800 Freyming-Merlebach (FR)**

(74) Mandataire: **Rieux, Michel**
**ELF ATOCHEM S.A.,**
**Département Propriété Industrielle,**
**4, Cours Michelet,**
**La Défense 10,**
**Cédex 42**
**92091 Paris la Défense (FR)**

(56) Documents cités:
EP-A- 0 268 999          EP-A- 0 445 859
US-A- 3 037 052

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

## Description

La présente invention concerne un procédé de fabrication d'acrylate de butyle secondaire par réaction, en présence d'une résine sulfonique, entre l'acide acrylique et les isomères de butène, pris individuellement ou en mélanges, tels qu'on les trouve, par exemple, dans les effluents gazeux générés par les unités de méthyl tertio-butyl éther (MTBE) ou d'éthyl tertio-butyl éther (ETBE).

La fabrication d'esters, plus particulièrement d'esters acryliques, par réaction entre une oléfine et un acide carboxylique est largement décrite dans la littérature. Les procédés mis en oeuvre diffèrent par l'état du milieu réactionnel (liquide-liquide, gaz-gaz, liquide-gaz), par le type de catalyse utilisé (homogène ou hétérogène), ou par l'état physique des réactifs (liquide ou gaz). Les catalyseurs utilisés en catalyse homogène sont souvent des acides minéraux forts.

L'utilisation de ces catalyseurs génère de nombreux problèmes, tels que corrosion des installations, formation en quantités importantes de sous-produits de type oligomères par exemple, séparations lourdes et difficiles engendrant des rejets acides.

Les difficultés de séparation du catalyseur peuvent être aisément résolues à l'aide de catalyseurs solides.

Les catalyseurs solides décrits dans la littérature diffèrent selon l'état des réactifs lors de la mise en réaction. Les plus souvent cités sont, dans le cas de contacts entre réactifs liquide/liquide ou liquide/gaz : des résines cationiques fortes macroporeuses, à groupements sulfoniques (US-A-3 037 052 ; US-A-2 678 332 ; EP-A-0 445 859) ; des zéolithes (US-A-4 365 084 ; US-A-4 448 983 ; JP-A-03 145 440) ; des sels du type n-dodécyl aza cycloheptan-2 one, HBr (WO 88/02361) ; et des sels de césium, rubidium, thallium de l'acide phosphotungstique (JP-A-04 139 149). L'utilisation des résines cationiques fortes macroporeuses, à groupements sulfoniques, est la plus souvent citée. Avec ces catalyseurs sélectifs qui n'engendrent pas de sous-produits colorés ou d'oligomères, comme c'est le cas avec les acides minéraux, on évite les problèmes de corrosion et de séparation catalyseur/produit brut de réaction.

Dans le brevet américain US-A-3 037 052, est décrite l'utilisation de résines cationiques sulfonées comme catalyseur dans la synthèse d'esters d'alkyles secondaires ou tertiaires. Est en particulier décrite la synthèse d'acrylate de butyle secondaire à partir de butène-1, avec un rapport molaire acide acrylique/butène-1 de 1/1.

A ce rapport molaire l'équilibre :

$$\text{Acide acrylique + Butène} \Leftrightarrow \text{acrylate de butyle secondaire}$$

n'est cependant pas suffisamment déplacé pour obtenir une très bonne conversion des réactifs mis en jeu. La présence d'acide acrylique résiduel à forte teneur dans le produit brut de réaction rend très complexe sa purification ultérieure par distillation, du fait du faible écart entre les températures d'ébullition de l'acide acrylique et de l'acrylate de butyle secondaire (respectivement de 141 et 131°C à 1,01 bar (760 mmHg) et de 95 et 85°C à 0,19 bar (150 mmHg)).

Pour éviter la présence d'acide acrylique à trop forte teneur dans le produit brut de réaction, il est préconisé, dans le brevet européen EP-B-0 445 859 d'effectuer la synthèse en utilisant un rapport molaire Butène/Acide acrylique supérieur à 1. En opérant ainsi en excès de butène, on obtient une très bonne conversion de l'acide acrylique et on a, de ce fait, une teneur minimum en acide acrylique dans le produit brut de réaction. Le rapport molaire optimal préconisé est compris entre 1 et 5, et c'est avec un rapport molaire de 3/1 que l'on obtient le meilleur compromis conversions/sélectivités.

En contrepartie, le fait d'opérer en excès de butène entraîne la formation de dimères de butènes (octènes), dont la séparation ultérieure d'avec l'acrylate de butyle secondaire par distillation pose d'énormes problèmes du fait du faible écart entre leurs températures d'ébullition (point d'ébullition de l'acrylate de butyle secondaire : 132°C / points d'ébullition des octènes : 112-122°C).

Devant les inconvénients qui viennent d'être décrits, liés à l'utilisation d'un rapport molaire butène/acide acrylique supérieur ou égal à 1, la Société déposante a découvert un moyen permettant de s'affranchir des inconvénients liés à l'utilisation des rapports molaires butènes/acide acrylique inférieurs à 1, autrement dit, un moyen permettant la présence d'acide acrylique résiduel à forte teneur dans le produit brut de la réaction de l'acide acrylique et des butènes en présence d'une résine sulfonique. Ce moyen est que l'acrylate de butyle secondaire distille, en présence d'eau, sous la forme d'un hétéroazéotrope facilement séparable de l'acide acrylique. A la pression de 0,19 bar (150 mmHg), les points d'ébullition de l'acide acrylique et de l'hétéroazéotrope acrylate de butyle secondaire/eau sont de respectivement 95 et 53,7°C, la composition massique de cet hétéroazéotrope étant, à cette pression, de 73,1% d'acrylate de butyle secondaire et de 26,9% d'eau.

Le fait d'opérer en excès molaire d'acide acrylique par rapport aux butènes présente, en dehors d'une moindre formation d'octènes difficilement séparables de l'acrylate de butyle secondaire, les avantages suivants : d'une part, il est possible de conduire le procédé dans des conditions de sécurité accrue par suite de la moindre concentration dans le milieu des butènes, qui sont des produits facilement inflammables, de la diminution du risque de montée en pression du réacteur en cas de polymérisation accidentelle et de l'élévation de température qui en résulterait, et de la plus grande maîtrise de l'introduction des butènes dans le réacteur ; d'autre part, la pression de service minimum requise pour maintenir les butènes en phase liquide est très inférieure à celle qu'il est nécessaire de mettre en oeuvre lorsqu'on opère avec un excès de butènes. La sécurité et la sélectivité vis-à-vis des octènes peuvent encore être accrues en introduisant les butènes sous forme de multi-injections.

La présente invention a donc pour objet un procédé de fabrication d'acrylate de butyle secondaire par réaction de l'acide acrylique et d'au moins un butène en présence d'une résine cationique forte macroporeuse, à groupements acide sulfonique, caractérisé par le fait que l'on conduit la réaction en utilisant un rapport molaire de l'acide acrylique au(x) butène(s) mis en oeuvre qui est supérieur à 1, le produit brut de réaction comprenant de l'acide acrylique résiduel, des butènes isomères dissous, l'acrylate de butyle secondaire recherché, des octènes, du butanol-2 et des produits lourds d'addition, et qu'on sépare l'acrylate de butyle secondaire recherché du produit brut de réaction.

Les butènes mis en jeu dans le procédé selon la présente invention sont le butène-1, le butène-2 cis, le butène-2 trans, pris seuls ou sous la forme de mélanges d'au moins deux d'entre eux. On peut également faire réagir des effluents gazeux (appelés Raffinats II) sortant des unités de fabrication de MTBE ou d'ETBE, et contenant une fraction de l'ordre de 50 - 90% en poids de butènes et une fraction de l'ordre de 10 - 50% en poids de butanes. Dans ce cas, les butanes resteront à l'état dissous dans le produit brut de la réaction et seront éliminés avec les butènes restants par le dégazage.

L'acide acrylique mis en jeu dans le procédé selon l'invention est aussi bien un acide acrylique glacial qu'un acide acrylique technique.

Conformément à l'invention, on utilise d'une manière générale un rapport molaire acide acrylique / butène(s) compris entre 1,05/1 et 10/1, de préférence, compris entre 1,5 et 3/1. L'utilisation de rapports molaires supérieurs à 3/1 est favorable à la conversion des butènes, mais est économiquement peu intéressante.

Les résines cationiques fortes macroporeuses à groupements acide sulfonique qui sont utilisables comme catalyseurs de la réaction conforme à la présente invention sont notamment celles ayant une capacité ionique comprise entre 0,6 et 2,5 éq./l, un diamètre de pores < 100 nm, et une surface spécifique comprise entre 40 et 100 $m^2$/g. On peut citer, entre autres, les résines commercialisées sous les dénominations AMBERLYST 15, LEWATIT SPC 112 ou 118. Ces résines sont utilisées à l'état aussi anhydre que possible. Lorsqu'elles sont achetées sous forme humide, elles doivent subir un séchage préalable, différents modes de séchage étant possibles tels qu'à l'aide d'un solvant sec de type alcool, en étuve ventilée à une température inférieure à 110°C, ou par distillation azéotropique. La teneur en eau maximum admissible dans le milieu réactionnel se situe à environ 0,5% en poids.

La réaction selon l'invention est généralement conduite à une température comprise entre 70 et 110°C. Il importe de ne pas dépasser la limite supérieure de 110°C afin de ne pas dégrader les résines sulfoniques. La réaction selon l'invention est exothermique ($\Delta H \sim 56,43$ kJ/mole). La valeur de la température de réaction est un compromis entre les contraintes thermodynamiques et les contraintes cinétiques. La plage de température préférée est comprise entre 80 et 95°C.

La réaction selon la présente invention est effectuée à la pression la plus basse possible compatible avec le maintien en solution des butènes. Celle-ci est comprise entre 8 et 20 bar et, préférentiellement, entre 8 et 12 bar. La pression n'a aucun effet sur la réaction ; elle ne sert qu'à assurer la mise en contact des réactifs.

La réaction est effectuée en présence d'au moins un inhibiteur de polymérisation, choisi notamment parmi l'hydroquinone et ses dérivés, l'éther monométhylique de l'hydroquinone, les phénols ayant des substituants stériquement encombrants, et la phénothiazine ; la teneur en inhibiteur(s) de polymérisation est généralement d'au moins 50 ppm par rapport à la charge acide acrylique + butène(s), les teneurs usuelles se situant entre 100 et 1000 ppm.

Le procédé selon la présente invention peut être conduit en discontinu ou en continu. Le temps de mise en réaction est généralement compris entre 1 et 5 heures, préférentiellement entre 1 et 2,5 heures, lorsqu'on opère en discontinu. En continu, le temps de passage est généralement compris entre 1 et 3 heures. Ces durées de réaction peuvent être accrues, mais cela se fait au détriment de la productivité.

Le produit brut de réaction contient de l'acide acrylique résiduel ; des butènes isomères dissous ; des butanes si l'on part de Raffinats II ; l'acrylate de butyle secondaire recherché ; des octènes ; du butanol-2 ; des produits d'addition de type acrylates en $C_8$, $C_{12}$, des produits lourds d'addition d'acide acrylique et d'acrylate de butyle secondaire, et d'addition de butanol-2 et d'acrylate de butyle secondaire.

Pour séparer l'acrylate de butyle secondaire recherché du produit brut de réaction, on peut procéder de la façon suivante :

Le produit brut de réaction est d'abord étêté afin d'en chasser les légers (butanes, butènes, ...), à une pression généralement comprise entre 0,001 bar et 0,665 bar (1 et 500 mmHg) et préférentiellement entre 0,066 bar et 0,133 bar (50 et 100 mmHg). Les octènes, le butanol-2 et les produits d'addition ci-dessus, sont ensuite éliminés par distillation à une pression généralement comprise entre 0,013 bar et 0,665 bar (10 et 500 mmHg), préférentiellement entre 0,066 bar et 0,266 bar (50 et 200 mmHg), tels quels ou sous forme d'azéotrope avec de l'eau. L'acide acrylique et l'acrylate de butyle secondaire sont séparés par distillation hétéroazéotropique en présence d'eau, à une pression de service généralement comprise entre 0,066 bar et 0,399 bar (50 et 300 mmHg), le rapport massique eau/alimentation de la colonne étant compris entre 20 et 150% et préférentiellement entre 50 et 100%.

**EP 0 745 579 B1**

Les exemples suivants illustrent la présente invention sans toutefois en limiter la portée. Dans ces exemples, les abréviations suivantes ont été utilisées :

- AA          = acide acrylique
- B1          = butène-1
- B2C         = butène-2 cis
- B2T         = butène-2 trans
- B           = butène 1 + butène-2 cis + butène 2-trans
- ABU II      = acrylate de butyle secondaire
- EMHQ        = éther méthylique de l'hydroquinone
- DIM         = octènes (les octènes, formés en petites quantités lors de la réaction selon l'invention, sont, pour l'essentiel, constitués de cis-5 méthyl heptène-3, trans-5 méthyl heptène-3, cis-3 méthyl heptène-3, et cis-3,4 diméthyl hexène-3)
- BuOH II     = butanol-2
- L1          = produit lourd d'addition AA + ABU II
- A.C8        = acrylate en $C_8$
- L2          = produit lourd d'addition BuOH II + ABU II

Exemples 1 à 3

Ces exemples mettent en évidence que la séparation AA/ABU II par distillation est rendue difficile par le fait que les points d'ébullition de ces produits sont très proches (Exemples 1 et 2). La distillation hétéroazéotropique en présence d'eau (Exemple 3) permet d'effectuer cette séparation très facilement.

Exemple 1

On conduit une distillation en continu, sous une pression de 0,066 bar (50 mmHg), d'un mélange AA/ABU II (composition massique : AA 55% - ABU II 45%) sur une colonne à garnissage Multiknit d'efficacité 15 plateaux théoriques (3 en épuisement - 12 en rectification).

Le débit de soutirage est régulé en fonction d'une consigne de température en tête de colonne.

Les conditions et résultats de cette distillation sont rapportés dans le Tableau 1 ci-après.

Exemple 2

On reproduit l'Exemple 1 en augmentant le taux de reflux et en abaissant la consigne de température en tête de colonne.

Les conditions et résultats de cette distillation sont également rapportés dans le Tableau 1.

## Tableau 1

| Exemple | Débit d'Alimentation de la Colonne (g/h) | Débit Tête (g/h) | Débit Pied (g/h) | Taux de Reflux | Température de Tête (°C) | Température de Pied (°C) | Composition Massique Tête | | Composition Massique Pied | | Rapport Massique ABU II Tête/ABU II Alimentation |
|---|---|---|---|---|---|---|---|---|---|---|---|
| | | | | | | | AA (%) | ABU II (%) | AA (%) | ABU II (%) | |
| 1 | 258 | 58 | 200 | 1,8/1 | 39 | 76,8 | 14 | 86 | 66 | 34 | 43 |
| 2 | 260 | 38 | 222 | 3,8/1 | 34 | 76 | 0,1 | 99,9 | 63 | 37 | 32 |

La majeure partie de l'ABU II se retrouve en pied, ce qui n'est pas très souhaitable. La qualité du produit pur est obtenue au détriment de la productivité.

<u>Exemple 3</u>

Le mélange AA/ABU II (composition massique : AA 50% - ABU II 50%) est séparé par distillation hétéroazéotropique en présence d'eau sous une pression de 0,19 bar (150 mmHg). La colonne utilisée est la même que dans les Exemples 1 et 2. L'ABU II distille sous forme d'un hétéroazéotrope avec l'eau, lequel est séparé dans un décanteur en deux phases : une phase organique $\phi_O$ et une phase aqueuse $\phi_A$, laquelle est renvoyée en reflux sur la colonne.

Les conditions et résultats de cette distillation hétéroazéotropique sont rapportés dans le Tableau 2 ci-après.

## Tableau 2

| Débit d'Alimentation de la Colonne (g/h) | Débit Reflux Phase Aqueuse (g/h) | Débit Tête (g/h) | Débit Pied (g/h) | Température Tête (°C) | Température Pied (°C) | Tête | | Pied | Rapport massique ABU II $\varnothing_o$ / ABU II Alimentation |
|---|---|---|---|---|---|---|---|---|---|
| | | | | | | $\varnothing_o$ Débit (g/h) | $\varnothing_A$ Débit (g/h) | | |
| 300 | 291 | 429 | 162 | 55 | 95,4 | 129 | 300 | | 85,3% |

Composition massique

| | $\varnothing_o$ | $\varnothing_A$ | Pied |
|---|---|---|---|
| AA (%) ................ | 0,09 | 0,1 | 88,8 |
| ABU II 5%) ............ | 99,2 | 1 | 11 |
| Eau (%) ............... | 0,7 | 98,9 | 0,2 |

L'utilisation de l'eau comme agent azéotrope facilite la séparation AA/ABU II et permet de minimiser la teneur en ABU II dans le pied de distillation.

Exemple 4

Dans un réacteur en acier inoxydable, de 250 ml, agité à l'aide d'un barreau magnétique, on introduit 72 g d'AA stabilisé avec 200 ppm d'EMHQ, 9 g de résine AMBERLYST 15 sèche, 29 g de B1, ainsi qu'un complément d'EMHQ dans une quantité telle que la teneur globale en EMHQ dans le milieu réactionnel soit de 700 ppm . Le rapport molaire AA/B1 est de 1,9/1.

On chauffe le mélange réactionnel à 90°C pendant 2 heures. La pression initiale autogène est comprise entre 7 et 8 bar. Elle est ajustée à 10 bar à l'azote, puis elle chute au fur et à mesure de l'avancement de la réaction.

A la fin de la réaction, la composition du produit brut de réaction est la suivante :

| Composition du produit brut de réaction | % en poids |
|---|---|
| AA | 42,6 |
| ABU II | 50,1 |
| Butènes | 5,6 |
| BuOH II | 0,03 |
| DIM | 0,8 |
| Lourds | 0,8 |

Les bilans pondéraux sont les suivants :

| | |
|---|---|
| Taux de conversion de l'AA | 41,2% |
| Taux de conversion de l'AA/B1 initial | 79% |
| Rendement | 76% |
| Sélectivité / AA | 95% |
| Sélectivité / B1 | 95% |

Exemples 5 à 8 (comparatifs)

On reproduit l'Exemple 4 en partant de différents isomères du butène, seuls ou en mélange, avec les rapports molaires Butène(s) / AA indiqués dans le Tableau 3, et en opérant la réaction à 80°C.

Les résultats sont également rapportés dans le Tableau 3.

Tableau 3

| Exemple Compa-ratif | Nature des butè-nes | Rapport molaire Butène(s)/AA | Analyse des gaz en fin d'essai (%)* | | | Taux ce conversion des butènes en DIM (%) |
|---|---|---|---|---|---|---|
| | | | B1 | B2C | B2T | |
| 5 | B1 | 3,4/1 | 9,5 | 27,5 | 61,2 | 25,6 |
| 6 | B2C | 3,4/1 | 5,5 | 29,2 | 63,8 | 25,6 |
| 7 | B1/B2T (57,1%-41,3%)* | 2,8/1 | 6,0 | 27,7 | 63,8 | 22,6 |
| 8 | B1/B2C/B2T (52,2%-19,7%-27,6%)* | 3,3/1 | 6,3 | 28,7 | 63,2 | 22,5 |

\* % donnés en poids

Les butènes s'isomérisent au contact de la résine lors de la réaction. En excès de butènes par rapport à la stoechiométrie AA/Butène(s), et quel que soit l'isomère de butène mis en jeu, on génère d'énormes quantités de DIM qui sont ensuite très difficilement séparables de l'ABU II par distillation.

Le Tableau 4 ci-après montre que les DIM se forment en moindres quantités dans le cas d'un rapport molaire AA/Butènes supérieur à 1, conformément à l'invention.

Exemples 9 à 11

On reproduit l'Exemple 4 en faisant varier le rapport molaire AA/B1. La température et le temps de mise en réaction sont respectivement de 90°C et 2 heures.

Les conditions réactionnelles et les résultats sont rapportés dans le Tableau 4.

Tableau 4

| Exemple | Rapport molaire AA/B1 | Composition du produit brut de réaction à la fin de la réaction (% en poids) | | | | | | Taux de conversion du réactif en défaut (%) | Rendement (%) | sélectivité /AA (%) | sélectivité /B1 (%) |
|---|---|---|---|---|---|---|---|---|---|---|---|
| | | AA | B | ABU II | DIM | A.C 8 | L1 | | | | |
| 9 (comparatif) | 0,33/1 | 3,5 | 22,1 | 57,3 | 15,1 | 0,2 | 0,2 | 90 | 91,4 | 99,7 | 60 |
| 10 (comparatif) | 1/1 | 18,7 | 9,3 | 65,9 | 2,8 | 0,2 | 2,2 | /AA:67 /B1:78 | /AA:64,8 /B1:67 | 96,4 | 85,3 |
| 11 | 3/1 | 43,3 | 3,8 | 50,9 | 0,7 | 0,03 | 0,4 | 84,3 | 78 | 97,4 | 93 |

Les résultats de l'Exemple Comparatif 9 confirment qu'un excès de B1 est pénalisant au niveau de la sélectivité par rapport à B1. On forme dans ce cas beaucoup de DIM, qu'il est ensuite difficile de séparer de l'ABU II.

Avec un rapport molaire AA/B1 > 1 (Exemple 11), on forme peu de DIM, et même encore beaucoup moins que dans le cas d'un rapport AA/B1 = 1 (Exemple Comparatif 10). La présence d'AA résiduel dans le produit brut n'est pas gênante dans la mesure où l'on peut, par la suite, le séparer de l'ABU II par distillation azéotropique en présence d'eau.

Exemple 12

On reprend l'Exemple 4 en remplaçant le butène-1 par un Raffinat II contenant :

- 34% en poids de butanes ;
- 10% en poids de B1 ;
- 36% en poids de B2T ; et
- 20% en poids de B2C

et en opérant dans les conditions suivantes :

| | |
|---|---|
| Température | 85°C |
| Rapport molaire AA/butènes | 1,9/1 |
| Pression initiale | 10 bar |
| Durée de réaction | 2 h |

A la fin de la réaction, la composition du produit brut est la suivante :

| Composition du produit brut de réaction | % en poids |
|---|---|
| AA | 34,1 |
| ABU II | 46,6 |
| Butènes + butanes | 18,3 |
| DIM | 0,8 |

Les bilans pondéraux sont les suivants :

| | |
|---|---|
| Taux de conversion de l'AA | 44% |
| Taux de conversion de l'AA/butènes initiaux | 83% |
| Rendement | 80,8% |
| Sélectivité/AA | 97,3% |
| Sélectivité/butènes | 95% |

Ces résultats sont conformes à ceux trouvés avec le butène-1 pur.

Exemples 13 à 17

On reproduit l'Exemple 4 en opérant à 80°C avec un rapport molaire AA/B1 de 2/1 et avec différentes résines du commerce. Les résines AMBERLYST sont commercialisées par la Société Rohm & Haas, et les résines LEWATIT, par la Société Bayer.

Les résultats sont rapportés dans le Tableau 5.

Tableau 5

| Exemple | Résines testées | Taux de conversion de l'AA(%) | Taux de conversion du butène (%) | Sélectivité par rapport à l'AA (%) | Sélectivité par rapport au B1 (%) |
|---|---|---|---|---|---|
| 13 | Amberlyst 15 | 47,5 | 79 | 99 | 99 |
| 14 | Lewatit SPC 112 | 39 | 66 | 80 | 92 |
| 15 | Lewatit SPC 118 | 29 | 51 | 91 | 94 |
| 16 | Amberlyst 39 | 35 | 62 | 91 | 97 |
| 17 | Amberlyst 36 | 35 | 63 | 95 | 92 |

Exemples 18 à 20

On conduit la réaction en continu à 80 - 85°C à partir du Raffinat utilisé à l'Exemple 12, avec un rapport AA/butènes de 2/1. Le temps de passage sur la résine est calculé par le rapport Volume de résine AMBERLYST 15 gonflée dans le milieu réactionnel / Débit de réactifs.

Les résultats sont rapportés dans le Tableau 6.

Tableau 6

| Exemple | Temps de passage (min.) | Composition du produit brut de réaction (% en poids) | | | | | Taux de conversion de l'AA | AA consommé/ Butènes initiaux | ABU II/AA initial | ABU II/AA consommé |
|---|---|---|---|---|---|---|---|---|---|---|
| | | AA | Butènes + Butanes | ABU II | DIM | Lourd AA + ABU II | | | | |
| 21 | 55 | 47 | 12 | 35,5 | 0,9 | 4,5 | 37 | 74 | 27 | 75 |
| 22 | 110 | 43,9 | 9,4 | 41 | 0,9 | 4,5 | 39 | 78 | 32 | 83 |
| 23 | 205 | 41 | 9,9 | 43,5 | 0,9 | 4,6 | 49,5 | 99 | 31,5 | 63 |

Les résultats obtenus en continu sont conformes à ceux obtenus en discontinu.

Exemples 24 à 27

On conduit la réaction en continu à 80 - 85°C à partir du butène-1, en faisant varier le rapport molaire AA/Butène-1, et le temps de passage sur la résine tel qu'il a été défini dans les Exemples 18 à 20.

Les conditions réactionnelles et les résultats sont rapportés dans le Tableau 7.

Tableau 7

| Exemple | Rapport Molaire AA/B1 | Temps de Passage (h) | Composition du Brut de réaction (% en poids) | | | | | | | Taux de conversion de l'AA (%) | Taux de conversion du B1 (%) | Butène converti en octènes / Butène-1 initial (%) | ABU II / AA consommé (%) |
|---|---|---|---|---|---|---|---|---|---|---|---|---|---|
| | | | AA | Butènes | ABU II | DIM | L1 | L2 | AC8 | | | | |
| 24 | 1,84 | 2 | 32,8 | 5,8 | 57,8 | 1,3 | 0,05 | 1,8 | 0,06 | 54 | 80,4 | 4,4 | 85,8 |
| 25 | 2,19 | 1,5 | 38,0 | 6,7 | 52,3 | 1,2 | 0,06 | 1,6 | 0,07 | 48,5 | 74,7 | 4,7 | 82,1 |
| 26 | 2,9 | 1,5 | 50,8 | 4,4 | 42,6 | 0,7 | 0,03 | 1,5 | 0,03 | 35,6 | 79,0 | 3,2 | 85,6 |
| 27 | 3,0 | 1 | 54,0 | 3,8 | 40,4 | 0,3 | 0,02 | 1,3 | 0,03 | 32,1 | 81,4 | 1,7 | 88,8 |

**Revendications**

1. Procédé de fabrication d'acrylate de butyle secondaire par réaction de l'acide acrylique et d'au moins un butène en présence d'une résine cationique macroporeuse à groupements acide sulfonique, caractérisé par le fait que l'on conduit la réaction en utilisant un rapport molaire de l'acide acrylique au(x) butène(s) mis en oeuvre qui est supérieur à 1, le produit brut de réaction comprenant de l'acide acrylique résiduel, des butènes isomères dissous, l'acrylate de butyle secondaire recherché, des octènes, du butanol-2 et des produits lourds d'addition, et qu'on sépare l'acrylate de butyle secondaire recherché du produit brut de réaction, par distillation azéotropique en présence d'eau.

2. Procédé selon la revendication 1, caractérisé par le fait qu'on utilise un rapport molaire acide acrylique/butène(s) compris entre 1,05/1 et 10/1.

3. Procédé selon la revendication 2, caractérisé par le fait qu'on utilise un rapport molaire acide acrylique/butène(s) compris entre 1,5 et 3/1.

4. Procédé selon l'une des revendications 1 à 3, caractérisé par le fait qu'on utilise, comme butène(s), du butène-1, du butène-2 cis, du butène-2 trans, ou un mélange d'au moins deux d'entre eux.

5. Procédé selon l'une des revendications 1 à 3, caractérisé par le fait qu'on utilise comme butène, un effluent gazeux obtenu en sortie de la fabrication de MTBE ou ETBE, ledit effluent contenant une fraction de l'ordre de 50 - 90% en poids de butènes et une fraction de l'ordre de 10 - 50% en poids de butanes, le produit brut de réaction comprenant alors également des butanes.

6. Procédé selon l'une des revendications 1 à 5, caractérisé par le fait qu'on utilise une résine cationique forte macroporeuse à groupements acide sulfonique qui a une capacité ionique comprise entre 0,6 et 2,5 éq./l, un diamètre de pores < 100 nm, et une surface spécifique comprise entre 40 et 100 $m^2$/g.

7. Procédé selon l'une des revendications 1 à 6, caractérisé par le fait qu'on conduit la réaction à une température comprise entre 70 et 110°C, de préférence, entre 80 et 95°C.

8. Procédé selon l'une des revendications 1 à 7, caractérisé par le fait qu'on conduit la réaction sous une pression comprise entre 8 et 20 bar, de préférence entre 8 et 12 bar.

9. Procédé selon l'une des revendications 1 à 8, caractérisé par le fait qu'il est conduit en discontinu avec un temps de mise en réaction compris entre 1 et 5 heures.

10. Procédé selon l'une des revendications 1 à 8, caractérisé par le fait qu'il est conduit en continu, avec un temps de passage compris entre 1 et 3 heures.

11. Procédé selon l'une des revendications 1 à 10, caractérisé par le fait que l'on conduit la réaction en présence d'au moins 50 ppm par rapport à la charge acide acrylique + butène(s), d'au moins un inhibiteur de polymérisation choisi parmi l'hydroquinone et ses dérivés, l'éther monométhylique de l'hydroquinone, les phénols ayant des substituants stériquement encombrants, et la phénothiazine.

**Claims**

1. Process for the manufacture of sec-butyl acrylate by reaction of acrylic acid and at least one butene in the presence of a macroporous cationic resin containing sulphonic acid groups, characterized in that the reaction is carried out using a molar ratio of the acrylic acid to the butene(s) used which is greater than 1, the crude reaction product comprising residual acrylic acid, dissolved butene isomers, the desired sec-butyl acrylate, octenes, 2-butanol and heavy addition products, and in that the desired sec-butyl acrylate is separated from the crude reaction product by azeotropic distillation in the presence of water.

2. Process according to Claim 1, characterized in that an acrylic acid/butene(s) molar ratio of between 1.05/1 and 10/1 is used.

3. Process according to Claim 2, characterized in that an acrylic acid/butene(s) molar ratio of between 1.5/1 and 3/1 is used.

4. Process according to one of Claims 1 to 3, characterized in that 1-butene, cis-2-butene, trans-2-butene or a mixture of at least two of these is used as butene(s).

5. Process according to one of Claims 1 to 3, characterized in that an effluent gas obtained after the manufacture of MTBE or ETBE is used as butene, the said effluent containing a fraction of about 50 - 90% by weight of butenes and a fraction of about 10 - 50% by weight of butanes, the crude reaction product then also comprising butanes.

6. Process according to one of Claims 1 to 5, characterized in that a macroporous strong cationic resin containing sulphonic acid groups which has an ionic capacity of between 0.6 and 2.5 eq/l, a pore diameter < 100 nm and a specific surface of between 40 and 100 $m^2$/g is used.

7. Process according to one of Claims 1 to 6, characterized in that the reaction is carried out at a temperature of between 70 and 110°C, preferably of between 80 and 95°C.

8. Process according to one of Claims 1 to 7, characterized in that the reaction is carried out under a pressure of between 8 and 20 bar, preferably of between 8 and 12 bar.

9. Process according to one of Claims 1 to 8, characterized in that it is carried out under batchwise conditions with a reaction time of between 1 and 5 hours.

10. Process according to one of Claims 1 to 8, characterized in that it is carried out continuously, with a throughput time of between 1 and 3 hours.

11. Process according to one of Claims 1 to 10, characterized in that the reaction is carried out in the presence of at least 50 ppm, relative to the acrylic acid + butene(s) charge, of at least one polymerization inhibitor chosen from hydroquinone and derivatives thereof, hydroquinone monomethyl ether, phenols having sterically bulky substituents, and phenothiazine.

## Patentansprüche

1. Verfahren zur Herstellung von *sek.*-Butylacrylat durch Umsetzung von Acrylsäure mit mindestens einem Buten in Gegenwart eines makroporösen kationischen Harzes mit Sulfonsäuregruppen, dadurch gekennzeichnet, daß die Umsetzung unter Anwendung eines Molverhältnisses von Acrylsäure zu eingesetztem Buten bzw. eingesetzten Butenen durchgeführt wird, das größer als 1 ist, wobei das Rohprodukt der Umsetzung nicht umgesetzte Acrylsäure, gelöste isomere Butene, das gewünschte *sek.*-Butylacrylat, Octene, 2-Butanol und hochsiedende Additionsprodukte enthält, und daß das gewünschte *sek.*-Butylacrylat durch azeotrope Destillation in Gegenwart von Wasser aus dem Rohprodukt der Umsetzung abgetrennt wird.

2. Verfahren nach Anspruch 1, dadurch gekennzeichnet, daß ein Molverhältnis Acrylsäure/Buten(e) im Bereich von 1,05/1 bis 10/1 verwendet wird.

3. Verfahren nach Anspruch 2, dadurch gekennzeichnet, daß ein Molverhältnis Acrylsäure/Buten(e) im Bereich von 1,5 bis 3/1 verwendet wird.

4. Verfahren nach einem der Ansprüche 1 bis 3, dadurch gekennzeichnet, daß als Buten bzw. Butene 1-Buten, *cis*-2-Buten, *trans*-2-Buten oder ein Gemisch von mindestens zweien dieser Butene verwendet wird.

5. Verfahren nach einem der Ansprüche 1 bis 3, dadurch gekennzeichnet, daß als Buten ein Gas verwendet wird, das am Ausgang der MTBE- oder ETBE-Herstellung anfällt, wobei das Abgas einen Mengenanteil von etwa 50 - 90 Gew.-% Butenen und einen Mengenanteil von etwa 10 - 50 Gew.-% Butanen enthält, wobei das Rohprodukt der Umsetzung dann außerdem Butane enthält.

6. Verfahren nach einem der Ansprüche 1 bis 5, dadurch gekennzeichnet, daß ein makroporöses stark kationisches Harz mit Sulfonsäuregruppen verwendet wird, das eine Ionenkapazität von 0,6 bis 2,5 Äquivalenten pro Liter, einen Porendurchmesser < 100 nm und eine spezifische Oberfläche von 40 bis 100 $m^2$/g aufweist.

7. Verfahren nach einem der Ansprüche 1 bis 6, dadurch gekennzeichnet, daß die Umsetzung bei einer Temperatur von 70 bis 110 °C, vorzugweise 80 bis 95 °C, durchgeführt wird.

8. Verfahren nach einem der Ansprüche 1 bis 7, dadurch gekennzeichnet, daß die Umsetzung bei einem Druck von 8 bis 20 bar, vorzugsweise 8 bis 12 bar, durchgeführt wird.

9. Verfahren nach einem der Ansprüche 1 bis 8, dadurch gekennzeichnet, daß es diskontinuierlich geführt wird und die Reaktionszeit von 1 bis 5 h beträgt.

10. Verfahren nach einem der Ansprüche 1 bis 8, dadurch gekennzeichnet, daß es kontinuierlich geführt wird mit einer Verweilzeit von 1 bis 3 h.

11. Verfahren nach einem der Ansprüche 1 bis 10, dadurch gekennzeichnet, daß die Reaktion in Gegenwart von mindestens 50 ppm, bezogen auf die eingesetzte Menge Acrylsäure + Buten(e), mindestens eines Polymerisationsinhibitors, der unter Hydrochinon und seinen Derivaten, Hydrochinonmonomethylether, Phenolen mit raumerfüllenden Substituenten und Phenothiazin ausgewählt wird, durchgeführt wird.